# EUROPEAN PATENT APPLICATION

(11) **EP 4 400 087 A1**
(43) Date of publication of application: **17.07.2024**
(21) Application number: 23315004.4
(22) Date of filing: 13.01.2023
(51) Int. Cl.: A61F 5/00

(54) **A DUODENAL ANCHOR FOR HOLDING IN PLACE AN IMPLANT IN A DUODENUM OF A PATIENT, AN IMPLANT FOR PLACEMENT IN A DUODENUM OF A PATIENT, A CONNECTOR FOR CONNECTING A DUODENAL ANCHOR AND A GASTRIC ANCHOR, AN IMPLANT FOR IMPLANTATION IN A PATIENT'S GASTRIC TRACT, COMPRISING A GASTRIC ANCHOR, A DUODENAL ANCHOR, AND A CONNECTOR.**

(71) Applicant: Baria Tek Medical, 75013 Paris (FR)
(72) Inventor: GRAY, Yonatan, 75011 Paris (FR); BIADILLAH, Youssef, 78600 Maisons-Laffitte (FR); NAZ, Christophe, 77300 Fontainebleau (FR); BAERD, Leonard Raphaël, 75018 Paris (FR)
(74) Representative: Hepp Wenger Ryffel AG

(57) **Abstract**

The invention relates to an implant system (500) for implantation in a patient's gastric tract. The system comprises a gastric anchor (400) adapted to be placed in a patient's stomach, a duodenal anchor (100), a connector (300) having a first (360) and a second end (370) and a central portion (310). The connector (300) is attached to the duodenal anchor (100) at a first end (360), and connected to the gastric anchor at the second end (370). The first (360) and second ends (370) of the connector (300) are connected by a central portion (310) and adapted, when the system (500) is implanted, to connect the gastric anchor (400) and the duodenal anchor (100) across a patient's pylorus.

## Description

The invention is directed to a duodenal anchor for holding in place an implant in a duodenum of a patient, an implant for placement in a duodenum of a patient, a connector for connecting a duodenal anchor and a gastric anchor, an implant system for implantation in a patient's gastric tract, comprising a gastric anchor, a duodenal anchor, and a connector, and methods of treating a patient according to the preamble of the independent claims.

Implants for placement in a patient's gastrointestinal tract, such as duodenum and/or stomach, are known in the prior art.

EP 3 878 415 discloses a device for treatment of obesity or diabetes comprising a duodenal tube and a first anchor. The first anchor is adapted for anchoring the tube distally to the pylorus without mucosal involvement. A second anchor in the form of a conical, inflatable balloon is used for anchoring the device in the stomach of the patient.

EP 4 062 879 discloses an anchor for anchoring a tube with respect to a pylorus of a patient. The anchor may comprise a gastric bulb which is collapsible for introduction and optionally self-expanding to an operative condition.

US 2007/250132 discloses devices and methods for applying gastrointestinal stimulation which include implanting a stimulation device including a body with at least one expandable portion and a bridging portion and at least one stimulation member in the gastrointestinal tract.

The devices according to the prior art do all have certain disadvantages. In particular, they may be difficult to deploy and/or anchor, they may create an undesirable mucosal engagement, they may have unreliable anchoring, and they may have an unreliable transfer of chyme.

It is an object of the present invention to overcome the drawbacks of the prior art, in particular to provide implants and duodenal anchors for holding in place an implant in a duodenum of a patient which is easier to manufacture, easier to deploy, which offers a reliable anchoring and which reduces negative side effects such as unreliable transfer of chyme.

A further object of the invention is to provide implants and treatments for placement in a gastric tract of a patient which are easy to manufacture, easy to deploy, which offer a reliable anchoring and which does not have negative side effects, in particular due to unreliable transfer of chyme.

These and other objects are achieved with devices and methods according to the characterizing portion of the independent claims. Further embodiments result from the dependent patent claims.

According to the invention, a duodenal anchor for holding in place an implant in a duodenum of a patient is provided. The duodenal anchor has an outer surface which is configured to adapt to an inner wall of the patient's duodenum. The duodenal anchor comprises an inner, preferably central, passage for conveying chyme. The duodenal anchor further comprises an inflatable part arranged at least partially circumferentially-with respect to the inner passage. The inflatable part, when fully filled with a fluid, has a larger circumference than an inner circumference of the duodenum.

The inflatable part may be a balloon.

Fully filled with fluid may be understood as being filled with a maximum quantity of fluid and/or gas without substantially increasing the pressure inside the inflatable part above the pressure outside the inflatable part (e.g. atmospheric pressure). Preferably, the inflatable part can accommodate a maximum of 10 ml to 60 ml in volume of fluid in its fully inflated state. More preferably, the inflatable part can accommodate a maximum of 25 ml to 45 ml in volume of fluid when fully inflated. In a preferred embodiment, the inflatable part may hold a volume of 34 ml when fully inflated.

This allows the inflatable part to reach the size of the duodenum without being fully inflated. As a result, the inflatable part may adapt to the inner surface of a duodenum, in particular its shape.

The inflatable part may be inflated by an incompressible fluid, such as a liquid (e.g. saline) and/or a gas (e.g. air). The use of air to inflate the inflatable part may result in a particularly soft structure when partially inflated.

A typical inner diameter of the human duodenum is 25 mm and a typical inner circumference of the duodenum 75 mm. The inflatable part, when fully inflated with a fluid and/or gas, may thus have a circumference larger than 75 mm and/or an inner diameter larger than 25 mm.

Preferably, the inflatable part has an outer diameter in the range from 30 mm to 70 mm, more preferably in the range from 35 mm to 55 mm, most preferably in the range from 35 mm to 45 mm, when fully inflated. In a preferred embodiment, the inflatable part has an outer diameter, when fully inflated, of 40 mm.

Preferably, the inflatable part is arranged circumferentially with respect to the inner passage which may be arranged centrally.

The outer surface of the duodenal anchor is configured to adapt to an inner wall of the patient's duodenum. To this end, the inflatable part may comprise a material which is mechanically flexible, such as a soft polymer. Additionally or alternatively, the inflatable part may be dimensioned such that at least an outer wall is mechanically flexible. The mechanical flexibility of the inflatable part, in particular its outer wall, may be sufficient to adapt to the inner wall of the patient's duodenum. The inflatable part and in particular the outer wall of the inflatable part may be made of silicone, e.g. silicone with a Shore A hardness of 30-60. The tensile strain of the inflatable part material and/or the outer wall material may be 100-300%.

The inner passage may have a diameter in the range from 10 mm to 25 mm, preferably from 15 mm to 21 mm, most preferably from 17 mm to 19 mm. In a preferred embodiment, the inner passage has a diameter of 18 mm.

The duodenal anchor according to the invention is particularly suited to be delivered to an implant site and partially inflated at the implant site. Therefore, the anchor may have a compressed shape which is compact and allows for easy and safe delivery. Particularly preferably, the inflatable part may be crimped under vacuum for delivery.,

Partial inflation, i.e. the filling of the inflatable part with an amount of fluid which does not lead to a fully filled state, may then be performed when the anchor is placed substantially at its final implant site. To this end, an inflation port may be present and in fluid communication with an inner volume of the inflatable part. A static syringe may be used to inflate the inflatable part. When the inflatable part is vacuum crimped, the fluid may infiltrate the inflatable part due to a pressure gradient and without additional pressure being provided on the syringe.

As the surrounding mucosa may constrain the inflatable part before the inflatable part reaches its nominal shape (i.e. before it is fully filled), the inflatable part may reach an equilibrium which is under-inflated. At this point, the inflation port may be closed and an inflation tube may be disconnected from a valve. As a result, the inflatable part reaches a shape which corresponds to the anatomy at the implant site, i.e. the anchor can be adjusted to a patient specific shape on-site.

Such a patient specificity is achievable with an anchor which has a circumference which is larger than an inner circumference of a typical human,duodenum because it allows for an under-inflated anchor to be securely implanted.

In general, the terms distal and proximal are used with respect to the direction of the gastrointestinal tract. A human stomach is at a proximal position with respect to the intestines. The intestines are at distal position with respect to a stomach. Accordingly, distal and proximal parts of any elements herein are meant to denominate their respective positions in an implanted position, i.e. oriented toward the stomach or intestines as described above.

In a preferred embodiment, the duodenal anchor has a cylindrical shape over at least part of its length. Preferably, the cylindrical shape extends over more than 40 % of its length. The length of the implant may be understood as an extension in a direction substantially parallel to a longitudinal axis of the duodenal anchor.

A cylindrical shape may in particular allow easy connection to other implants, for example a duodenal tube.

In a preferred embodiment, the duodenal anchor comprises a valve mechanism connected or connectable to the inflatable part for filling the inflatable part.

The valve mechanism may be connected to an inflation port. The valve mechanism may be adapted to receive an inflation tube which may open the valve mechanism when it is inserted. The valve mechanism may further be adapted to close when such a tube is removed.

In general, when the valve mechanism is in an open state, fluid may flow into the inflatable part from an outside source which is connected or connectable to the inflation port and/or the valve mechanism. When the valve mechanism is closed, no fluid may exit the inflatable part through the valve mechanism.

In a preferred embodiment, the duodenal anchor is attached to a proximal end of a sleeve, the sleeve having a distal end for placement within the small intestine.

In a preferred embodiment, the inner passage of the duodenal anchor is substantially tube shaped.

The inner passage of the duodenal anchor may be substantially tube shaped over at least 60 percent, more preferably 80 %, most preferably 90 % of its length along a direction substantially parallel to a longitudinal axis of the implant body.

In a preferred embodiment, the inflatable part of the duodenal anchor comprises a mechanically flexible outer wall.

In a state where the duodenal anchor with the inflatable part is placed in a duodenum of a patient, the inflatable part is only partially filled. As a result of being only partially filled, the mechanically flexible outer wall of the inflatable part may have an amorphic shape.

The mechanically flexible outer wall may provide several advantages. First, in a radial direction, it may succumb to the peristaltic motion of the bowels trying to push the outer wall of the inflatable part back and forth. Second, in an axial direction, it may enable the mechanically flexible outer wall to have an amorphic shape, resulting from the partial inflation of the inflatable part. As a result, only fluid within the inflatable part-may be moved back and forth, as a consequence of peristaltic motion of the bowels, instead of the whole duodenal anchor.

In a preferred embodiment, the duodenal anchor, preferably the inflatable part, is crimped together by a release mechanism, preferably a outer jacket and a release wire, to reduce a radial size of the duodenal anchor for delivery. Particularly preferably, the outer jacket comprises or consists of silicone.

In order to facilitate delivery of the duodenal anchor inside the duodenum of a patient, it is advantageous to reduce the size, especially the radial size of the duodenal anchor.

The inflatable part may be put under vacuum and crimped together by a release mechanism. The release mechanism may comprise a silicone outer jacket tied together by a release wire adapted such that by pulling on an end of the release wire the outer jacket releases the pressure exercised by it on the duodenal anchor.

In a preferred embodiment, the duodenal anchor comprises elongated elements with free distal ends that are extending in a direction substantially parallel to the longitudinal axis of the duodenal anchor.

The distal end of the duodenal anchor may be facing the small intestine when implanted in a patient's duodenum.

The elongated elements may be made of a same or different material as the duodenal anchor. The elongated elements preferably comprise or consist of a soft material and may, additionally or alternatively, be dimensioned to be mechanically flexible (i.e. deform elastically under typical forces exerted in an implanted state). Elongated elements may provide stability to, e.g., a sleeve which is placed circumferentially around the elongated elements. It will be understood that the elongated elements may substantially have the same function as the elongated elements according to a preferred embodiment of the implant according to the invention. When the elongated elements are attached to the duodenal anchor, this may provide a particularly simple treatment because no separate implant needs to be provided.

Preferably, the elongated elements are arranged around the end of the inner passage at the distal end of the duodenal anchor. When extending along a direction parallel to a longitudinal axis of the duodenal anchor, the elongated elements preferably form a structure having the shape of a tube with straight longitudinal cuts. Preferably, each cut has the same width as each of the elongated elements.

In a preferred embodiment, the duodenal anchor is connectable, at a proximal end, to one of a connector and a stent, preferably by a plurality of connection elements arranged at a circumferential position with respect to the central passage.

Preferably, the duodenal anchor comprises at least one channel extending through a wall which is adapted for receiving a connector.

The proximal end of the duodenal anchor is facing the stomach when implanted in a patient's duodenum. The distal end of the duodenal anchor is opposite the proximal end.

Where a gastric anchor formed as a balloon is used to treat a patient, the duodenal anchor may also be connected or connectable to a gastric balloon.

According to another aspect of the invention, an implant for placement in a duodenum of a patient is provided. The implant having a generally tube-shaped implant body with a proximal end and a distal end. The proximal end of the implant body is fixable or fixed to a duodenal anchor, preferably a duodenal anchor as previously described. The implant body comprises a plurality of elongated elements with free distal ends extending in a direction substantially parallel to a longitudinal axis of the implant body. The free ends of the elongated elements form a distal-most portion of the implant body.

Preferably, the proximal end of the implant body is fixable or fixed to a duodenal anchor, more preferably to the distal end of the duodenal anchor. The distal end of the duodenal anchor may be facing the small intestine when implanted in a duodenum of a patient.

The implant body may be generally tube-shaped over at least 60 percent, more preferably 80 %, most preferably 90 % of its length along a direction substantially parallel to a longitudinal axis of the implant body.

The generally tube-shaped implant body together with the elongated elements may have a castle-like shape.

Circumferential spaces between two adjacent elongated elements may form a passage for chyme as the elongated elements will bend and kink with the anatomy of the patient. The circumferential spaces between the elongated elements may allow reliable transfer of chyme even when the elongated elements are bent and/or kinked, for example due to the anatomy of the patient. As a result, an chyme channel formed by the tube-like structure is less prone to blocking due to e.g. kinking.

Preferably, the width of each circumferential space between the longitudinal elements equals the width of each one of the elongated elements.

The circumferential spaces may be gaps.

The length of each elongated element may be between 5 mm and 30 mm, preferably between 10 mm and 15 mm.

Preferably, each elongated element has the shape of a rectangular cuboid. Preferably, the width of the elongated elements is larger than the thickness of the elongated elements. Preferably, the length of the elongated elements is larger than both, the width and the thickness of the elongated elements.

Preferably, the elongated elements comprise or consist of the same material as the implant body. Additionally or alternatively, the elongated elements comprise or consist of a mechanically softer material that the implant body.

Preferably, the elongated elements comprise or consist of durable thermoplastic such as thermoplastic polyurethane (TPU) and/or silicone. The material may have a Shore A hardness of 60.

Preferably, the elongated elements are arranged around a circle with the same diameter as the inner passage forming an extension of the tube-like shape.

Each of the elongated elements may have a width between 2 and 15 mm, preferably between 4 and 8 mm, particularly preferably between 5 and 6 mm.

In an advantageous embodiment, the implant further comprises a, preferably intestinal, sleeve arranged around the tube-shaped implant body and attached to the proximal end of the implant body, wherein a distal end of the sleeve is configured for placement within the small intestine. The sleeve may be adapted for transport of chyme and be adapted to reduce or prevent contact of chyme with an intestine wall.

The sleeve may be placed at a distance of 60 to 100 cm of the proximal bowels, i.e. the sleeve may cover the duodenum and parts of the jejunum. The diameter of the sleeve may be between 15 and 30 mm, preferably between 20 and 25 mm.

In an advantageous embodiment, the implant body comprises 3 to 20, preferably 6 to 12, elongated elements.

In an advantageous embodiment, the implant comprises or consists of a soft material, preferably an elastic soft material.

Preferably the elongated.elements and the implant body comprise or consist of the same material.

Particularly preferably, the elongated elements consist of a soft material, preferably an elastic soft material, such as medical grade silicone.

The material may be a material with a Shore A hardness of less than 55-65.

In an advantageous embodiment, the implant has an outer dimension, in a direction perpendicular to a longitudinal axis, between 1 and 10 cm, preferably between 10 and 30 mm.

In an advantageous embodiment, the elongated elements have a closed surface, in particular in a direction perpendicular to a longitudinal axis.

A closed surface may be understood as being solid elements in the sense that they are filled with material, i.e., for example, they do not have holes through them.

For example, an elongated element having the shape of a rectangular cuboid, where the elongated element is filled with material (i.e. no holes), exhibits four closed surfaces in a direction perpendicular to a longitudinal axis.

Preferably, the elongated elements of the implant exhibit a closed surface in a direction perpendicular to a longitudinal axis, whereas the closed surface is the largest surface of the elongated element.

In an advantageous embodiment, at least one circumferential space, preferably all circumferential spaces, between the elongated elements of the implant body have a width corresponding to the width of the elongated elements. Where not all elongated elements have the same width, the width of the space may correspond to the width of at least one elongated element.

In an advantageous embodiment, the elongated elements of the implant body are impermeable to liquids and gases.

Impermeable to liquids and gases means that it does not allow the passage of liquids and gases or at least reduces the rate at which liquids or gases can pass through.

In an advantageous embodiment, the generally tube-shaped implant body has walls without openings and/or has a substantially contiguous surface.

The elongate elements may in particular have a straight shape without curves along the longitudinal axis. The elongate elements may consist of a soft material and/or of the same material as the implant body.

A solid rectangular cuboid may form a wall without openings.

According to still another aspect of the invention, a connector for connecting a duodenal anchor and a gastric anchor is provided. Preferably, the duodenal anchor is a duodenal anchor as previously described. The connector has a central portion and a first and second plurality of extremities arranged at opposite ends of the central portion. The central portion has a generally elongated shape along a longitudinal axis. The first plurality of extremities is configured to be fixed or fixable to the duodenal anchor at first extremity free ends. The second plurality of extremities is configured to be fixed or fixable to the gastric anchor at second extremity free ends. The first plurality and second plurality free ends extend away, in a radial direction, from the longitudinal axis. Preferably, the first plurality and second plurality free ends have connecting means for connection to a duodenal anchor and a gastric anchor, respectively.

The connector according to the invention allows to connect two elements, such as the gastric anchor and the duodenal anchor, through a pylorus while still allowing chyme to pass therethrough.

The gastric anchor may be a stent or an inflatable gastric anchor such as a balloon. The inflatable gastric anchor may be fully inflated.

Where the gastric anchor is inflatable, the inflatable part may have a nominal volume of 30 to 80 ml, preferably 50 to 60 ml.

Where the gastric anchor is a stent, a stent length may be in the range of 15 to 50 mm, preferably 20-30mm. A diameter of the stent may be in the range of 30 to 80 mm, preferably 50 to 60 mm. The stent may comprise 9-18 stent cells, preferably 12-15.

Wires of the stent may extend in the pylorus with a length of 5 to 50 mm, preferably 20 to 30 mm. The number of wires may be between 2 and 6, preferably between 3 and 4.

The tensile elasticity of the wires may be up to 300%, whereas the assembled wires (which typically may include three wires which may also be glued or tied to duodenal and/or gastric anchors), the tensile elasticity is preferably in the range of 20 to 100%, particularly preferably 60 to 80%.

The gastric anchor may have a length to diameter ratio of 1:5 - 1:1, preferably 1:2.

The duodenal anchor may be an inflatable or a non-inflatable duodenal anchor. The duodenal anchor may also comprise inflatable parts and non-inflatable parts.

Preferably, the central portion has a generally elongated shape and extends in a parallel direction along the longitudinal axis of the connector.

Preferably, the first plurality of extremities is configured to extend inside the duodenal anchor, whereas it may be casted in the duodenal anchor.

Preferably, the second plurality of extremities is configured to extend inside the gastric anchor and may be cast in the gastric anchor.

In a particularly preferred embodiment, at least one of the first and second plurality of extremities of the connector is formed by rods, preferably exactly three rods.

Preferably, the rods of the first plurality of extremities extend through the central portion and are connected to, or form, the second plurality of extremities.

Preferably, the first and/or second plurality of extremities is formed by 2 to 6 rods, more preferably by 3 to 4 rods.

Preferably, the rods of the first and second plurality of extremities have a tensile elasticity of 20% to 100%, more preferably of 60% to 80%.

In a particularly preferred embodiment, at least one of the first plurality free ends and the second plurality free ends of the connector are arranged at substantially constant angles and/or radial distances with respect to the longitudinal axis.

The first and/or second plurality free ends may all end on a virtual circle that is centered and normal to the longitudinal axis.

In a particularly preferred embodiment, the central portion of the connector, in a direction along the longitudinal axis, extends over a length between 3 and 50 mm, preferably between 20 mm and 30 mm. Generally, the central portion may have a length which is at least the length of a typical pyloric valve and may preferably have a length which is shorter than twice the typical pyloric valve.

In a particularly preferred embodiment, the lengths of the first plurality of extremities and/or the second plurality of extremities of the connector is between 10 and 50 mm, preferably between 20 and 30 mm.

These lengths allow the connector to partially extend beyond the pylorus valve and thus reduces or avoids abrasion of anchor shafts against the pylorus.

In some embodiments, the distance between the first and second extremities in a direction along a longitudinal axis is 20 to 30 mm.

In a particularly preferred embodiment, the first plurality of extremities and/or the second plurality of extremities and/or the central portion of the connector comprise or consist of a soft material.

A soft material is a material having a low hardness and a high flexibility. For example, silicone and/or polyurethane may be suitable materials. Preferably, the soft material has a tensile elasticity of 20% to 100%, more preferably of 60% to 80%. Preferably, the soft material has a Shore A hardness between 40 and 70, more preferably between 55 and 65.

In a particularly preferred embodiment, the central portion of the connector is formed by a plurality of central extensions and a circumferentially arranged tubular element. The central portion may be formed by a plurality of central extensions and a tubular element (350) which is arranged circumferentially around the central extensions. The tubular element may have a length at least as long as its diameter. The tubular element may be configured to reduce the lateral diameter of the central portion and/or such as to bring the central extensions into physical contact with each other.

The length of the tubular element may be measured along a direction parallel to a longitudinal axis of the tubular element and may have the same length as the central portion. Preferably, the tubular element has a length between 3 and 50 mm, more preferably between 5 and 30 mm.

Preferably, the tubular element has a wall thickness between 0.2 and 4 mm, more preferably between 0.5 and 2 mm.

Preferably, the tubular element comprises or consists of the same material as the first and/or second plurality of extremities and/or the central portion of the connector. The tubular element may comprise or consist of medical silicone.

The plurality of central extensions may be 2 to 6 rods connected or connectable to the first and second plurality of extremities.

The plurality of central extensions may comprise a smaller number of central extensions than the first and/or second plurality of extremities comprises extremities.

Each central extension of the plurality of central extensions may be connected or connectable to more than one.extremity of the first plurality of extremities.

Each central extension of the plurality of central extensions may be connected or connectable to more than one extremity of the second plurality of extremities.

Preferably, the tubular element is configured to surround all elements of the central portion. More preferably, the tubular element is configured to surround the plurality of central extensions.

According to still another aspect of the invention, an implant system for implantation in a patient's gastric tract is provided. The implant system comprises a gastric anchor, preferably one of a stent and a balloon, adapted to be placed in a patient's stomach. The implant system further comprises a duodenal anchor, preferably a duodenal anchor as previously described. The implant system further comprises a connector, preferably a connector as previously described. The connector has a first and a second end and a central portion. The connector is connected or connectable to the duodenal anchor at a first end, and connected or connectable to the gastric anchor at the second end. The first and second ends of the connector are connected by a central portion. The connector is adapted, when the system is implanted, to connect the gastric anchor and the duodenal anchor across a patient's pylorus.

It will be understood that in some embodiments it is conceivable to use a non-inflatable duodenal anchor.

Preferably, the connector is adapted to connect the gastric anchor and the duodenal anchor. The connector may already connect the gastric anchor and the duodenal anchor before the system is implanted, or they may be connected during a surgical treatment, e.g. when in a patient.

The length of the duodenal anchor may be 20 to 80 mm, preferably 50 to 60 mm, in along a direction in which chyme goes through the duodenal anchor/system. The length may include elongated elements as part of the duodenal anchor and/or an implant body with elongated elements which is connect to the duodenal anchor.

The gastric anchor, whether formed as a stent or a balloon, may have length in the range of 15 to 50 mm, preferably 20-30mm. A diameter of the gastric anchor may be in the range of 30 to 80 mm, preferably 50 to 60 mm. Where the gastric anchor is formed by a balloon, the nominal volume of the gastric anchor may be 40 ml.

The length of the connector, which is adapted to extend across the pylorus, may be between 5 and 50 mm, preferably 20 to 30 mm.

In a preferred embodiment, the implant system further comprises an implant, preferably an implant as previously described. The implant comprises an implant body. The implant body may be tube shaped. The implant body may be attached to the duodenal anchor at a proximal end. Preferably, the implant body comprises elongated elements extending in a direction away from the duodenal anchor.

In a preferred embodiment, the implant system further comprises a sleeve arranged around the tube-shaped implant body. The sleeve is attached to the proximal end of the implant body, whereas a distal end of the sleeve is configured for placement within the small intestine.

According to still another aspect of the invention, a method of treating a patient comprising the implantation of an implant system as previously disclosed is provided.

The method of treating a patient may comprise any one of the following steps:
- deposition of a guide wire in the proximal duodenum via an endoscope work channel;
- insertion of a concentric delivery system loaded with the implant system as previously disclosed having the distal tip of the delivery system ending with an atraumatic ball, over the deposited guide wire;
- pushing the delivery system through the patient throat and esophagus until reaching the stomach with the portion having the loaded docking station such that the ball and some of the sleeve may be distal to the pylorus;
- inserting an endoscope into the stomach for imaging purposes;
- under visual guidance of the endoscope, advancing the docking station forward, having the duodenal member passing the pylorus;
- pushing the sleeve distally via an internal catheter at the rear end of the delivery system, until sleeve is fully deployed in the small intestines;
- release of the distal atraumatic ball;
- injecting pressurized fluid through the sleeve via the internal catheter while retrieving the catheter. Sleeve is to reach full patenc, which may be confirmed via X-ray imaging;
- release of a duodenal anchor crimp sleeve and partial inflation to -desired volume
- un-sheath a gastric stent in the antrum (distal stomach) if the the system comprises a gastric anchor formed by a stent or inflate the gastric anchor to its nominal volume if the gastric anchor is formed by a balloon;
- disconnection of balloons once anchor position and deployment is visually confirmed via the endoscope;
- retrieval of the endoscope;
- retrieval of the delivery system.

According to still another aspect of the invention, a method of treating a patient is disclosed. The method of treating a patient comprises the step of placing a duodenal anchor comprising an inflatable part, preferably a duodenal anchor as previously described, in a patient's duodenum. The method of treating a patient further comprises a step of filling a predetermined quantity of fluid into the inflatable part. The predetermined quantity of fluid is between 10% and 80% of a volume of the inflatable part when fully inflated. The inflatable part may accommodate, when fully inflated, a volume of 10 ml to 60 ml, preferably, 25 ml to 45 ml, particularly preferably 34 ml.

In a preferred embodiment, the predetermined quantity of fluid of the method of treating a patient is between 5 and 10 ml. However, it will be understood that any amount of liquid smaller than 50 ml, preferably less than 20 ml, particularly preferably less than 10 ml, may be suitable, depending on the size of the duodenal anchor that is used, and may lead to only partial inflation. In some embodiments, 1 to 5 ml are used.

The invention is now described with reference to certain embodiments and figures which show:
Figure 1: schematic representation of a first embodiment of a duodenal anchor.
Figure 2: schematic representation of a second embodiment of a duodenal anchor.
Figure 3: schematic representation of an embodiment of the first embodiment of a duodenal anchor connected to an implant.
Figure 4: schematic representation of an embodiment of an implant.
Figure 5: Schematic representation of an embodiment of a connector.
Figure 6: Schematic representation of an embodiment of the connector connected to a duodenal anchor.
Figure 7: Schematic representation of an embodiment of an implant system.
Figure 8: Representation of another embodiment of the implant system.
Figure 9: Schematic representation of another embodiment of the implant system.
Figure 10: representation of an embodiment of the duodenal anchor crimped by a release mechanism.
Figure 11: representation of an embodiment of the duodenal anchor in a deflated state.

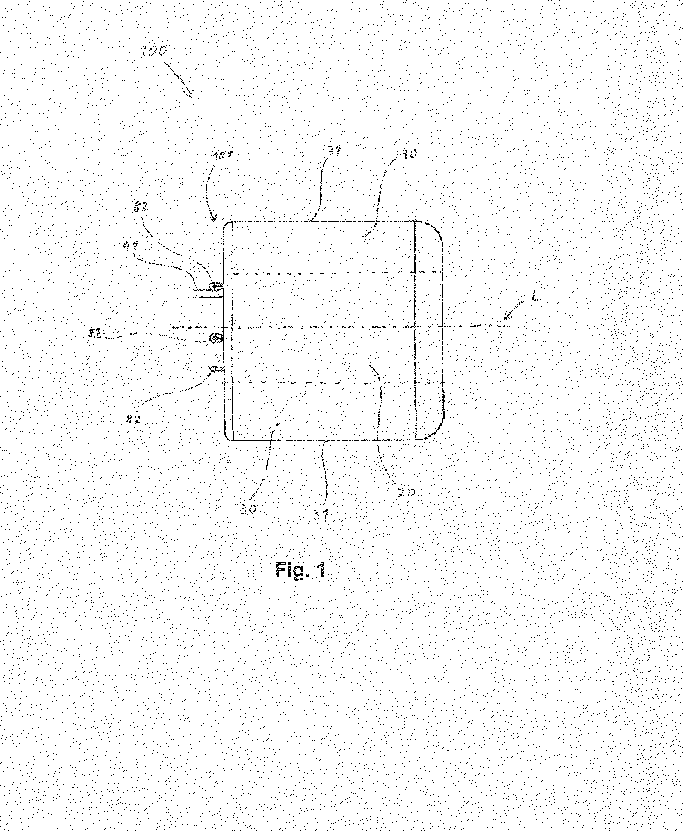

Figure 1 shows a first embodiment of a duodenal anchor 100. The duodenal anchor 100 comprises an inner passage 20 for conveying chyme, which is illustrated by two dotted lines. The inner passage 20 has the shape.of a tube and is centered around a longitudinal axis L of the duodenal anchor 100. An inflatable part 30 is arranged around the inner passage 20. The inflatable part 30 has a mechanically flexible outer wall 31 that is configured to adapt to an inner wall of the patient's duodenum. The inflatable part 30 is connected to a valve mechanism 41. The duodenal anchor 100 has a proximal end 101 with connection elements 82 which are adapted to connect to e.g. a connector and/or a gastric anchor (not shown). The duodenal anchor shown here consists of silicone.

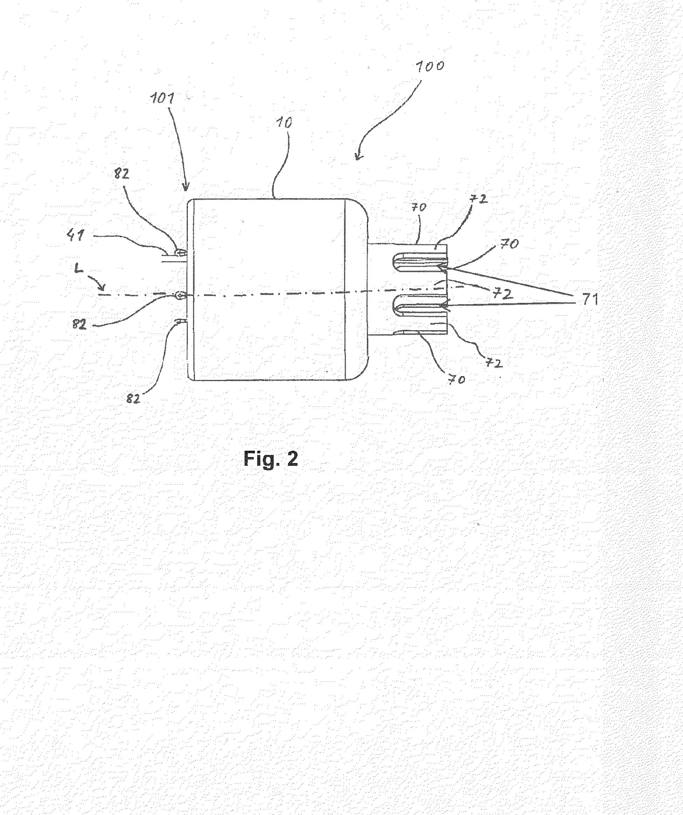

Figure 2 shows an further embodiment of a duodenal anchor 100. The duodenal anchor 100 is similar to the embodiment shown in Fig. 1. For clarity, identical features are not described separately here. Here, the duodenal anchor 100 is made of polyurethane and further comprises elongated elements 70 with free ends 72 and separated by gaps 71 between the free ends. The duodenal anchor 100 has a mechanically flexible outer wall 10 that is configured to adapt to an inner wall of the patient's duodenum.

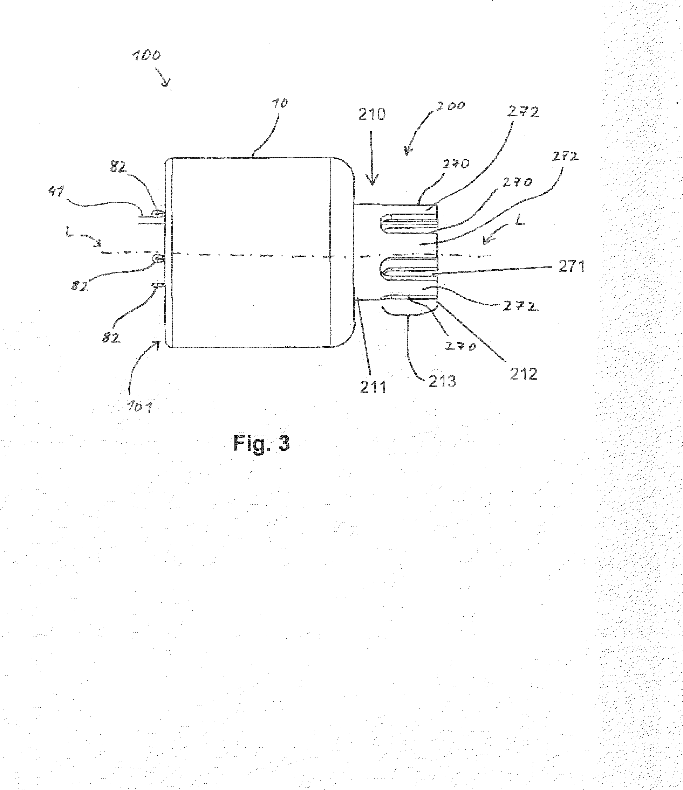

Figure 3 shows a schematic representation of an implant 200 connected to a duodenal anchor 100 as shown in Fig. 1. The implant 200 has a tube-shaped implant body 210 with a proximal end 211 and a distal end 212. The proximal end 211 is attached to the duodenal anchor 100. A plurality of elongated elements 270 with free distal ends 272 extend in a direction substantially parallel to the longitudinal axis L. The plurality of elongated elements 270 form a distal-most portion 213 of the implant body 210. Between the elongated elements 270, in a direction following the circumference of the implant body 210, there is a circumferential space 271. The duodenal anchor 100 has a mechanically flexible outer wall 10 that is configured to adapt to an inner wall of the patient's duodenum.

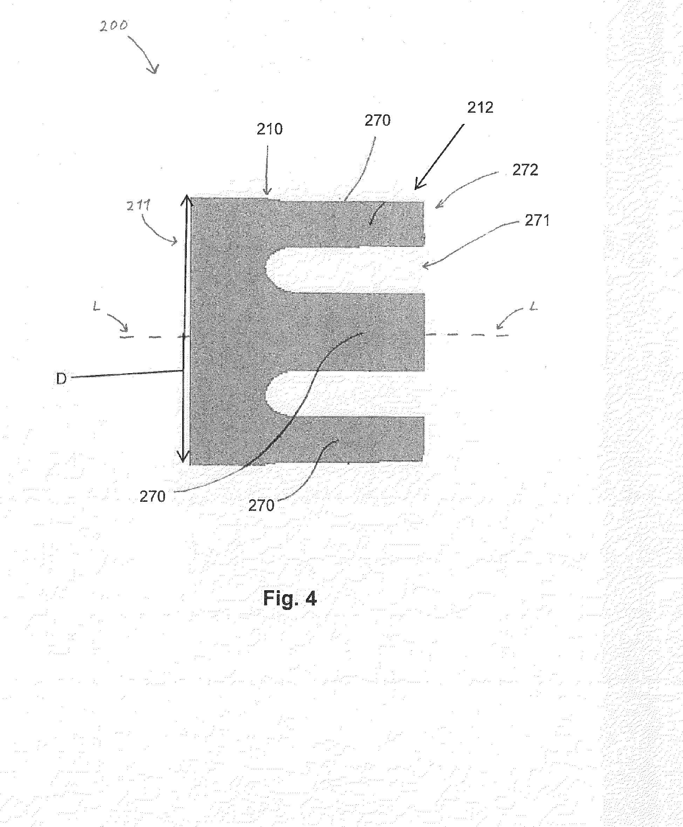

Figure 4 schematically shows an implant 200 according to the invention substantially formed by an implant body 210. At a proximal end 211, the implant body is formed substantially as a tube with closed lateral surface. From the proximal end 211 in a generally distal direction along the longitudinal axis L(from left to right as shown in Fig. 4), elongated elements 270 with free distal ends 272 are formed and separated by gaps 271 therebetween. The elongated elements 270 substantially form the distal end 212 of the implant body 210. The implant is made of medical-grade silicone. The implant body 210 shown here has an outer dimension D, in a direction perpendicular to the longitudinal axis L, of 5 cm. Here, the elongated elements 270 have closed surfaces and do not have any holes or discontinuities. The elongated elements are substantially solid and integrally formed extensions.

It will be understood that the implant of Fig. 4 combined with the duodenal anchor of Fig. 1 may result in a device which is functionally similar to the embodiment of Fig. 2 which is integrally formed.

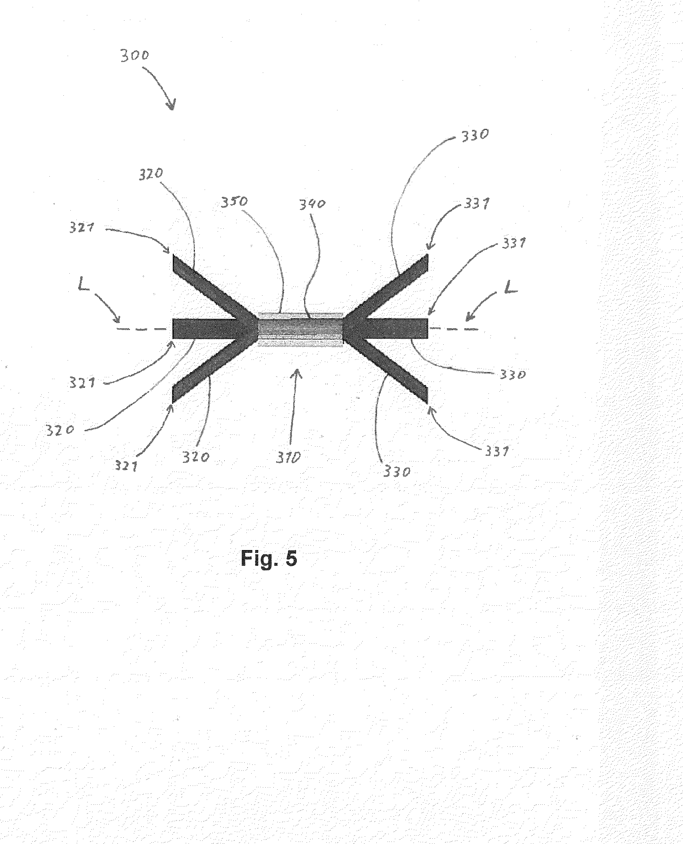

Fig. 5 shows a schematic representation of an embodiment of a connector 300. The connector 300 has a central part 310, a first plurality of extremities 320 and a second plurality of extremities 330. Preferably, the first plurality of extremities 320 and the second plurality of extremities 330 are formed by rods. The first and second plurality of extremities 320, 330 are terminated by first and second free ends 321, 331, respectively. The first plurality of extremities 320 and the second plurality of extremities 330 are connected by a plurality of central extensions 340, which are formed integrally in the present example, that extend throughout the central part 310. A tubular element 350 is circumferentially arranged around the central extensions 340 in the central part 310. The tubular element 350 extends over the whole length of the central part 310 and its length is considered in a direction along the longitudinal axis L of the connector 300. The plurality of central extensions 340 may, additionally or alternatively, be glued together in the central part.

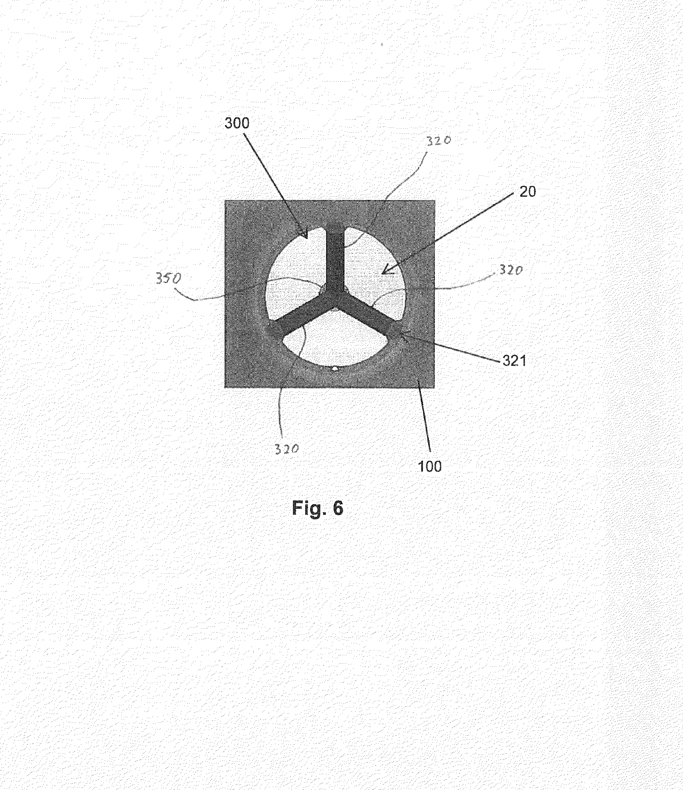

Figure 6 shows a schematic representation of an embodiment of the connector 300 connected to the duodenal anchor 100. Shown is a view along the longitudinal axis of the connector (see Fig. 5). The first plurality of extremities 320 of the connector 300 extend from the central part (not shown) surrounded by the tubular element 350. The first extremity free ends 321 are connected to the duodenal anchor 100 at a circumferential positions of the inner passage 20 at the proximal end of the duodenal anchor 100. The first plurality of extremities 320 are arranged at substantially constant angles and radial distances with respect to the longitudinal axis of the connector.

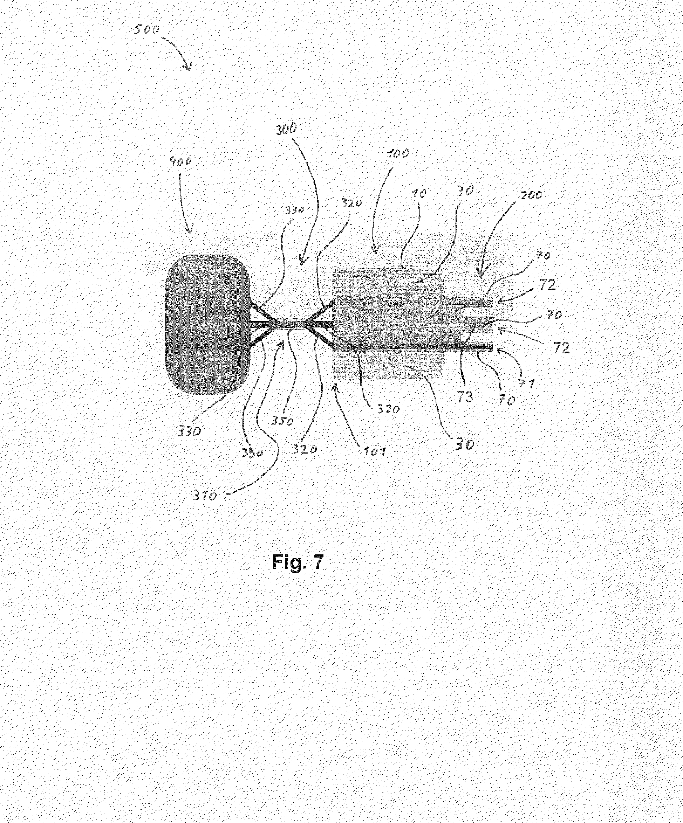

Figure 7 shows a schematic representation of an embodiment of an implant system 500. The implant system 500 comprises a gastric anchor 400, a duodenal anchor 100 having an inflatable part 30, similar to the one shown in Fig. 1, a connector 300 similar to the one shown in Fig. 5, and an implant 200 similar to the implant shown in Fig. 4. The implant 200 comprises elongated elements 70 with free distal ends 72. The elongated elements 70 have closed surfaces 73, i.e. the elongated elements 70 are filled with material. The implant 200 is connected to the distal end of the duodenal anchor 100. The distal end of the duodenal anchor is on the opposite side of the proximal end 101 of the duodenal anchor 100. The duodenal anchor 100 has an outer surface 10 which is configured to adapt to an inner wall of the patient's duodenum. At the proximal end 101 of the duodenal anchor 100, the first plurality of extremities 320 of the connector 300 are connected to the duodenal anchor 100. From the proximal end 101 of the duodenal anchor 100 towards the central part 310, the first plurality of extremities 320 are pulled together due to the tubular element 350. The second plurality of extremities 330 of the connector 300 are connected to the gastric anchor 400. The gastric anchor 400 shown here is an inflatable balloon, but may be replaced by a stent in some embodiments.

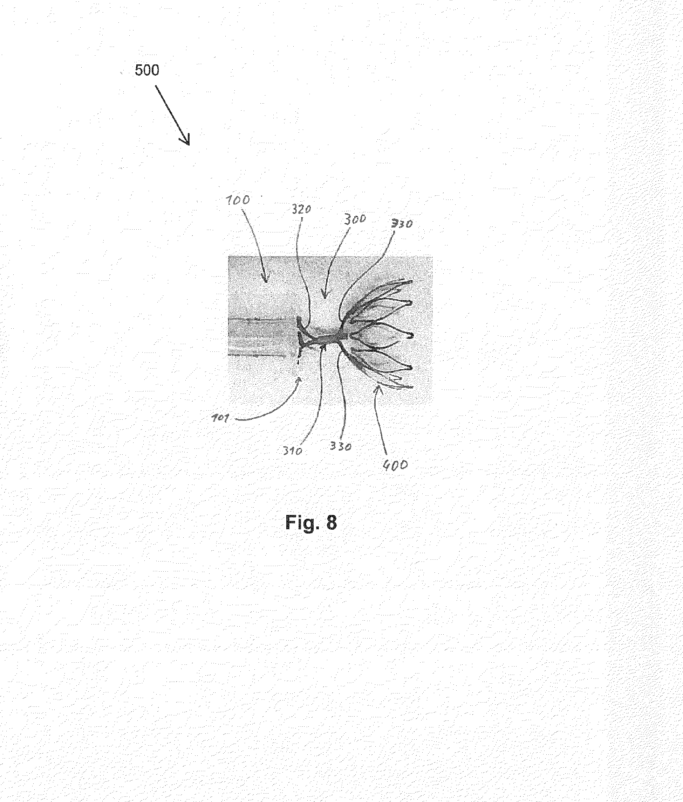

Figure 8 shows a representation of another embodiment of the implant system 500. The duodenal anchor 100 is connected through the connector 300 to the gastric anchor 400 which is formed by a stent. Other parts of the system substantially correspond to the embodiment shown in Fig. 7 and identical parts are not described again for clarity.

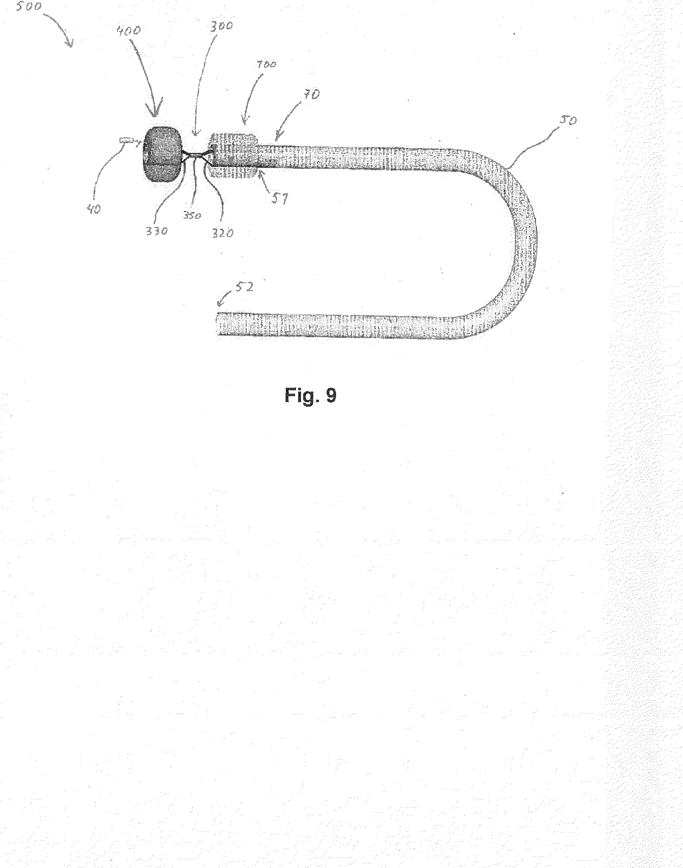

Figure 9 shows a schematic representation of another embodiment of the implant system 500. The gastric anchor 400 is formed as a balloon and connected through the connector 300 to the duodenal anchor 100 comprising elongated elements 70. The elongated elements are arranged inside a sleeve 50. The gastric balloon 400 comprises a valve mechanism 40 for inflation. The duodenal anchor is inflatable by a separate valve mechanism (not shown, see Figs. 2 and 3). Additionally or alternatively, the valve mechanism 40 may also be connected to the inflatable part of the duodenal anchor for inflating said inflatable part. The sleeve 50 has a distal end 52 and a proximal end 51. The proximal end 51 of the sleeve 50 is connected to the duodenal anchor 100 such that the elongated elements 70 are inside the sleeve 50 at the proximal end 51 of the sleeve 50.

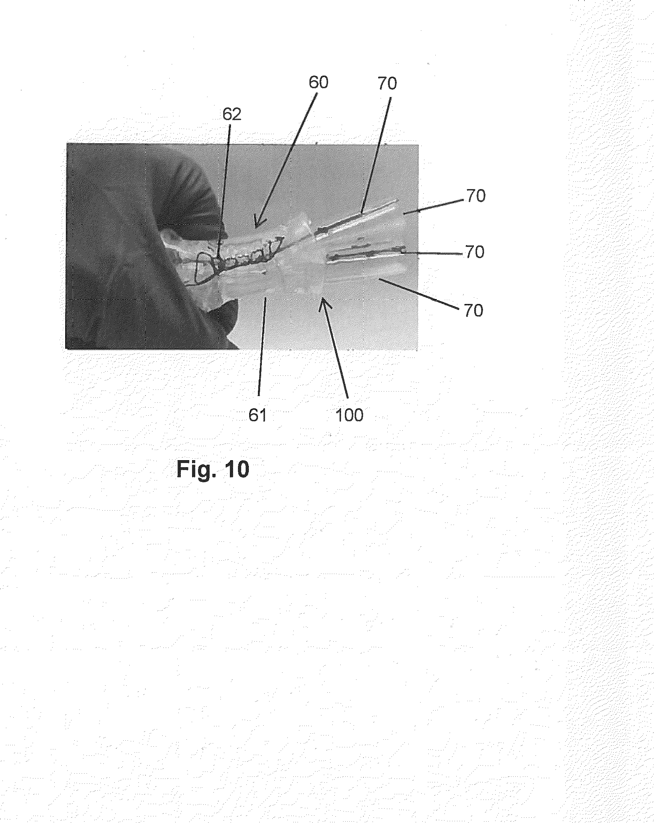

Figure 10 shows a duodenal anchor 100 according to the invention in a crimped state. The duodenal anchor 100 is crimped by a release mechanism 60 comprising a silicone outer jacket 61 and a release wire 62. The silicone outer jacket 61 of the release mechanism 60 is placed around the duodenal anchor 100, is pulled together such that it applies a concentric force to the duodenal anchor 100 and kept in this configuration by the release wire 62. Release wire can be selectively released, independent of inflation, by an operator such as to expand the duodenal anchor 100.

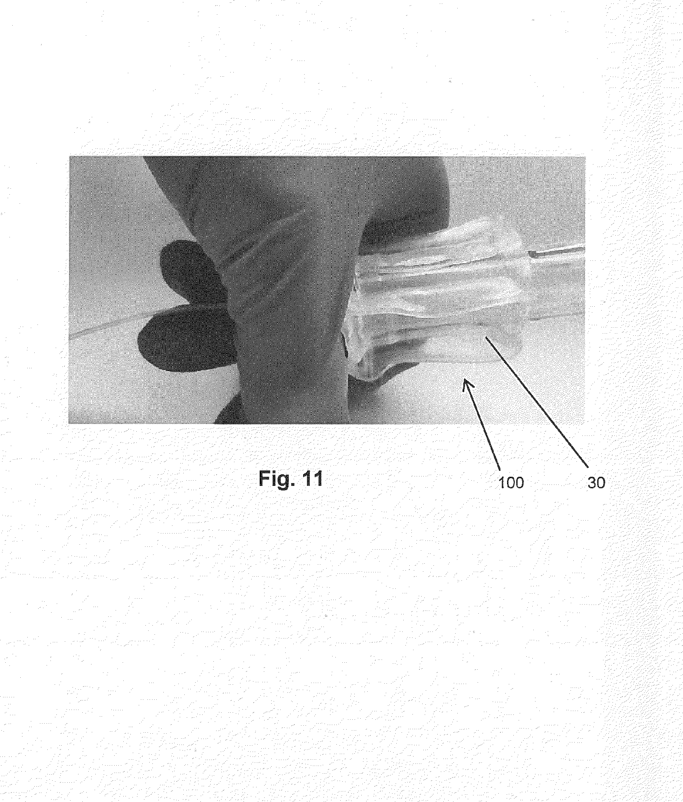

Figure 11 shows the duodenal anchor 100 of Fig. 10 after release of the release wire and removal of the silicone jacket (both not shown, see Fig. 10). The inflatable part 30 of the duodenal anchor 100 is in a deflated state, i.e. there is no or very few liquid and/or gas inside the inflatable part 30 of the duodenal anchor 100.

## Claims

1. A duodenal anchor (100) for holding in place an implant in a duodenum of a patient, having an outer surface (10, 31) which is configured to adapt to an inner wall of the patient's duodenum, the duodenal anchor (100) comprising:
- an inner, preferably central, passage (20) for conveying chyme, and
- an inflatable part (30) arranged at least partially circumferentially with respect to the inner passage (20), **characterized in that** the inflatable part (30), when fully filled with a fluid, has a larger circumference than an inner circumference of the duodenum.

2. The duodenal anchor (100) according to claim 1, wherein the duodenal anchor (100) has a .cylindrical shape over at least part of its length, preferably over more than 40 % of its length, in a direction substantially parallel to a longitudinal axis (L) of the duodenal anchor (100).

3. The duodenal anchor (100) according to claim 1 or 2, wherein the duodenal anchor (100) comprises a valve mechanism (40, 41) connected or connectable to the inflatable part (30) for filling the inflatable part (30).

4. The duodenal anchor (100) according to any one of the preceding claims, wherein the duodenal anchor (100) is attached to a proximal end (51) of a sleeve (50), the sleeve having a distal end (52) for placement within the small intestine.

5. The duodenal anchor (100) according to any one of the preceding claims, wherein the inner passage (20) is substantially tube shaped.

6. The duodenal anchor (100) according to any one of the preceding claims, wherein the inflatable part (30) comprises a mechanically flexible outer wall (31).

7. The duodenal anchor (100) according to any one of the preceding claims, wherein the duodenal anchor (100), preferably the inflatable part (30), is crimped together by a release mechanism (60), preferably a outer jacket(61) and a release wire (62), particularly preferably a silicone outer jacket, to reduce a radial size of the duodenal anchor (100) for delivery.

8. The duodenal anchor (100) according to any one of the preceding claims, wherein the duodenal anchor (100) comprises elongated elements (70, 270) with free distal ends (72, 272) that are extending in a direction substantially parallel to the longitudinal axis (L) of the duodenal anchor (100) .

9. The duodenal anchor (100) according to any one of the preceding claims, wherein the duodenal anchor (100) is connectable, at a proximal end (101), to one of a connector (300) and a stent (400), preferably by a plurality of connection elements (82) arranged at a circumferential position with respect to the central passage (20).

10. An implant (200) for placement in a duodenum of a patient, having a generally tube-shaped implant body (210) with a proximal end (211) and a distal end (212), wherein the proximal end (211) is fixable or fixed to a duodenal anchor, preferably a duodenal anchor (100) according to any one of the preceding claims, and wherein the implant body (210) comprises a plurality of elongated elements (220) with free distal ends (272) extending in a direction substantially parallel to a longitudinal axis (L) of the implant body (210), such that the free ends (72, 272) of the elongated elements (70, 270) form a distal-most portion of the implant body (210).

11. The implant (200) according to claim 10, wherein the implant (200) further comprises a sleeve (50) arranged around the tube-shaped implant body (210) and attached to the proximal end (211) of the implant body (210), wherein a distal end (52) of the sleeve (50) is configured for placement within the small intestine.

12. The implant (200) according to claim 10 or 11, wherein the implant body(210) comprises 3 to 20, preferably 6 to 12, elongated elements (70, 270).

13. The implant (200) according to claims 10 to 12, wherein the implant (200) comprises or consists of a soft material, preferably an elastic soft material.

14. The implant (200) according to claims 10 to 13, wherein the implant (200) has an outer dimension (D), in a direction perpendicular to a longitudinal axis (L), between 1 and 10 cm.

15. The implant (200) according to claims 10 to 14, wherein the elongated elements (70, 270) have a closed surface (73), in particular in a direction perpendicular to a longitudinal axis (L)

16. The implant (200) according to claims 10 to 15, wherein at least one circumferential space (271) between the elongated elements (70, 270) has a width corresponding to the width of the elongated element (70, 270).

17. The implant (200) according to claims 10 to 16, wherein the elongated elements (70, 270) are impermeable to liquids and gases.

18. The implant (200)according to claims 10 to 17, wherein the generally tube-shaped implant body (210) has walls without openings.

19. A connector (300) for connecting a duodenal anchor (100) and a gastric anchor (400), preferably wherein the duodenal anchor (100) is a duodenal anchor (100) according to any one of claims 1 to 9,
having a central portion (310) and a first (320) and second plurality of extremities (330) arranged at opposite ends of the central portion (310), the central portion (310) having a generally elongated shape along a longitudinal axis (L)
wherein the first plurality of extremities (320) is configured to be fixed or fixable to the duodenal anchor (100) at first extremity free ends (321), wherein the second plurality of extremities (330) is configured to be fixed or fixable to the gastric anchor (400) at second extremity free ends (331),
wherein the first plurality (321) and second plurality free ends (331) extend away, in a radial direction, from the longitudinal axis (L) and preferably have connecting means for connection to a duodenal anchor (100) and a gastric anchor (400), respectively.

20. The connector (300) according to claim 19, wherein at least one of the first (320) and second plurality of extremities (330) is formed by rods, preferably exactly three rods.

21. The connector (300) according to claim 19 or 20, wherein at least one of the first plurality free ends (321) and the second plurality free ends (331) are arranged at substantially constant angles and/or radial distances with respect to the longitudinal axis (L).

22. The connector (300) according to claims 19 to 21, wherein the central portion (310), in a direction along the longitudinal axis (L), extends over a length between 3 and 50 mm, preferably between 20 and 30.mm.

23. The connector (300) according to claims 19 to 22, wherein the lengths of the first plurality of extremities (320) and/or the second plurality of extremities (330) is between 10 and 50 mm, preferably between 20 and 30 mm.

24. The connector (300) according to claim 19 or 23, wherein the first plurality of extremities (320) and/or the second plurality of extremities (330) and/or the central portion (310) comprise or consist of a soft material.

25. The connector (300) according to any one of claims 19 to 24, wherein the central portion (310) is formed by a plurality of central extensions (340) and a tubular element (350) arranged circumferentially around the central extensions, preferably such as to bring the central extensions (340) into physical contact with each other.

26. An implant system (500) for implantation in a patient's gastric tract, comprising
a gastric anchor (400), preferably one of a stent and a balloon, adapted to be placed in a patient's stomach,
a duodenal anchor (100), preferably a duodenal anchor (100) according to any one of claims 1 to 9,
a connector (300), preferably a connector (300) according to any one of the claims 19 to 25, having a first (360) and a second end (370) and a central portion (310),
wherein the connector (300) is attached to the duodenal anchor (100) at a first end (360), and connected to the gastric anchor at the second end (370),
further wherein the first (360) and second ends (370) of the connector (300) are connected by a central portion (310) adapted to be placed, when the system (500) is implanted, across a patient's pylorus to connect the gastric anchor (400) and the duodenal anchor (100).

27. The system (500) according to claim 26, further comprising an implant (200), preferably an implant (200) according to 10 to 18, with an implant body (210), wherein the implant body (210) is attached to the duodenal anchor (100) at a proximal end (101) and preferably wherein the implant body (210) comprises elongated elements (70) extending in a direction away from the duodenal anchor (100).

28. The system (500) according to any one of claims 26 to 27, further comprising a sleeve (50) arranged around the tube-shaped implant body (210) and attached to the proximal end (211) of the implant body, wherein a distal end of the sleeve (50) is configured for placement within the small intestine.

29. A method of treating a patient comprising the implantation of a system (500) according to claims 26 to 28.

30. A method of treating a patient, comprising the steps of placing a duodenal anchor (100) comprising an inflatable part (30), preferably a duodenal anchor (100) according to claims 1 to 9, in a patient's duodenum, further comprising a step of filling a predetermined quantity of fluid into the inflatable part (30), wherein the predetermined quantity is between 10% and 80% of a volume of the inflatable part when fully inflated.

31. The method of treating a patient according to claim 30, wherein the predetermined quantity is between 5 and 10 ml.
